Europäisches Patentamt

European Patent Office

Office européen des brevets

(11) Publication number: 0 204 522
A2

## (12) EUROPEAN PATENT APPLICATION

(21) Application number: 86304118.2

(22) Date of filing: 30.05.86

(51) Int. Cl.4: **C07K 15/00** , G01N 33/577 , C12P 21/00 , C12N 15/00 , G01N 33/569 , ///(C12P21/00,C12R1:91)

(30) Priority: 30.05.85 US 740124

(43) Date of publication of application: 10.12.86 Bulletin 86/50

(84) Designated Contracting States: CH DE FR GB IT LI NL SE

(71) Applicant: GENETIC SYSTEMS CORPORATION 3005 First Avenue Seattle Washington 98121(US)

(72) Inventor: Nelson, Karen A. 4927 S.W. Admiral Way Seattle Washington 98116(US) Inventor: Antonelli, Paolo Via Paggiolette 2, Apt. C. I-54100 Massa(IT) Inventor: Hansen, John A. 8416 S.E. 80th St. Mercer Island Washington 98040(US)

(74) Representative: Harrison, David Christopher et al MEWBURN ELLIS & CO 2/3 Cursitor Street London EC4A 1BQ(GB)

(54) Monoclonal antibody panel for histocompatibility typing.

(57) Novel hybrid cell lines, the monoclonal antibodies prepared therefrom and their use in diagnostics and therapy are described, where said antibodies bind to human alloantigens.

Except where otherwise indicated, the murine hybridomas listed in Tables 1 and 2 were deposited at the American Type Culture Collection and given accession numbers provided for in Tables 1 and 2.

EP 0 204 522 A2

## MONOCLONAL ANTIBODY PANEL FOR HISTOCOMPATIBILITY TYPING

The major histocompatibility complex (MHC) in man is denoted HLA and is encoded by a series of linked loci located on the short arm of chromosome 6. Class I HLA antigens are encoded by the HLA-A, B, and C loci while class II antigens are encoded by the HLA-DR, DP and DQ loci.

The HLA antigens are highly polymorphic, i.e., there is a large number of alleles at each locus. Because of this high degree of polymorphism, the determination of an individual's "HLA type" conventionally requires testing of that individual's cells with a large panel of antibodies to identify which two alleles are present at each locus. An individual's HLA type, then, is the sum of the alleles assigned for each locus.

The HLA antigens are known to play a major role in the acceptance or rejection of cells or tissues transplanted between individuals (Klein, Immunology: The Science of Self-Nonself Discrimination, John Wiley, New York, 1982). The likelihood of acceptance of the transplanted cells or tissue is increased when the recipient and the donor share HLA alleles. The transplantation of different tissues requires a different stringency of matching. For example, platelet transfusion requires less stringency than bone marrow transplantation.

The HLA antigens are also known to play a role in disease susceptibility. The presence of particular alleles results in a predisposition to certain diseases. For example, the presence of the B27 allele is so strongly correlated with ankylosing spondylitis as to be useful in the differential diagnosis of this disease.

The high degree of polymorphism of the HLA antigens also makes them particularly useful for paternity testing.

HLA polymorphisms have been largely defined by complement-dependent cytotoxicity assays, using sera from multiparous women. These sera have a number of limitations. They are often weak and multispecific, and those directed to some of the rarer polymorphisms are difficult to find. Thus it is necessary to screen large numbers of sera in order to find one or a few which are useful. Furthermore, because of the comparatively small volumes usually available, it is necessary to continually replace these sera with new sera which must be standardized and checked against the previous ones.

Deliberate immunization of human volunteers for the purpose of producing alloantisera has proved useful, but has obvious practical and ethical limitations. In view of these problems, several attempts have been made to produce xenogeneic antisera recognizing HLA polymorphisms. However, such xenoantisera have been found to contain predominantly antibodies to species-specific determinants; hence extensive absorption is required to detect antibodies to polymorphic determinants - (Staines et al., Tissue Antigens (1973) 3:1; Goodfellow et al., ibid (1976) 7:105). Some success in raising antisera which recognize polymorphisms has been achieved by using the combined strategies of immunizing with purified material and immunizing species which are closely related evolutionarily (Barnstable et al., Cell (1979) 14:9; Sanderson et al., Transplantation (1973) 16:304). Nonetheless, extensive absorptions are still usually required to remove the non-polymorphic activity of these antisera.

With the advent of the hybridoma technique of Kohler and Milstein, Nature (1975) 256:495, it was anticipated that it would be relatively straightforward to produce potent and specific anti-HLA reagents and thereby circumvent the problems discussed above. Most of the monoclonal anti-HLA antibodies so far produced, however, have recognized either monomorphic or broadly cross-reacting determinants and hence have had limited utility in tissue typing. In fact, based on the information obtained heretofore, there has remained substantial uncertainty whether or not a significant number of HLA encoded alloantigens possess unique epitopes and whether experimental animals with the appropriate Ir gene repertoire for responding to those epitopes will be available, should such distinct epitopic sites exist. There is also uncertainty whether monoclonal antibodies which detect useful specificity groups can be developed.

Brief Description of the Prior Art

Brodsky et al., Immunological Review, (1979) 47:4, provides an excellent review of the field at the time of the article. Grumet et al., Human Immunology (1982) 5:61, describes a monoclonal antibody which divides HLA-B27 into two subgroups. Muller et al., Human Immunology (1983) 6:189, describes an IgM monoclonal antibody directed against an antigenic determinant which is shared by the HLA allospecificities A2 and A28. Muller et al., Human Immunology (1983) 7:229, describes a monoclonal antibody specific for the private alloantigenic determinant of the HLA-B13 molecule. Antonelli et al., Human Immunology (1984) 11:11, describes a monoclonal antibody, P8.1, which recognizes the human alloantigen, HLA-B8. The antibodies P8.1, P5.1, P5.3 and P5.4, which are described herein, were used in the Ninth International Histocompatibility Workshop and Conference, Munich, West Germany, May 6-11, 1984 and published in "Histocompatibility Testing 1984," ed. Albert, Bauer and Mayr, Springer-Verlag, Berlin, 1984. See also, EPA 0 068 790, 0 073 953; 0 103 368; 0 131 878; and PCT/US81/01684.

## SUMMARY OF THE INVENTION

Methods and compositions are provided which permit histocompatibility typing of human lymphoid cells. Hybrid cell lines are produced which secrete monoclonal antibodies specific for various HLA alloantigens. Panels of these monoclonal antibodies find use in diagnosis and therapy. The hybrid cell lines can also serve as sources of DNA encoding antibodies for the various HLA allospecificities, which DNA can be manipulated using recombinant DNA (rDNA) technology to enable other cellular hosts to produce specific antibody.

## DESCRIPTION OF THE SPECIFIC EMBODIMENTS

In accordance with the subject invention, novel cell lines and compositions are provided, which allow for histocompatibility typing of human cells, particularly lymphoid cells. The subject cell lines have a murine chromosome, wherein the germ-line DNA has been rearranged to code for an antibody receptor(s) having a binding site(s) specific for an epitope(s) on an HLA antigen(s). Panels of these monoclonal antibodies produced by the subject cell lines can be used in a wide variety of methods, both for diagnosis and therapy.

The monoclonal antibodies of this invention are prepared by immortalizing nucleic acid capability for expression of antibodies specific for the various human HLA alloantigens. Immortalization may be achieved by introducing DNA encoding for the antibody(ies) into a host capable of being grown in culture or by transformation of a cell capable of antibody production. The immortalized cell line may be a mammalian cell line which has been transformed through oncogenesis, by transfection, mutation or the like. Such cells include myeloma lines, lymphoma lines, etc., namely those lines which are capable of supporting the expression and secretion of the antibody(ies) in vitro (tissue culture). The antibody may be a naturally occurring immunoglobulin of a mammal, produced by transformation of a lymphocyte, particularly a splenocyte by means of a virus or by fusion of a lymphocyte with a neoplastic cell, for example, a myeloma, to produce a hybrid cell line. Alternatively, the antibody(ies) may be an immunoglobulin produced by rDNA techniques, where, for example, genomic DNA or cDNA coding for the heavy and light chain of an HLA-specific monoclonal antibody is inserted into an expression vector for expression of the chains. The transformation or fusion can be carried out in conventional ways, the fusion being described in an extensive number of U.S. Patent Nos. including: 4,172,124; 4,350,683; 4,363,799; 4,381,292; and 4,423,147; see also Kennett et al., "Monoclonal Antibodies", New York: Plenum Press, 1980, and references cited therein; and transformation being described in, for example, U.S. Patent No. 4,464,465. The hybrid cell lines may be cloned and screened in accordance with conventional methods to detect antibodies specific for each of the various HLA alloantigens. Usually, each hybridoma line is established by at least three cycles of cloning by limiting dilution. The cells producing the desired antibody are initially identified by conventional assays (e.g., cytotoxicity assays) and then expanded and screened for specificity and sensitivity employing a large number - (>250) of human blood donors. The appropriate hybrid cell lines may be expanded in culture or injected into the peritoneal cavity of an appropriate host for the production of ascites fluid.

By virtue of having antibody(ies) specific for one or more HLA polymorphic determinants, the supernatants from candidate hybridomas may be screened in competition with the subject monoclonal antibodies known to be specific for the site in a competitive assay. Thus hybrid cell lines can be readily produced from a variety of sources based on the availability of antibodies specific for a particular polymorphism. Alternatively, where hybrid cell lines are available which produce antibodies specific for the subject polymorphism, these hybrid cell lines may be fused with other neoplastic B cells where such other B cells may serve as recipients for genomic DNA encoding the monoclonal antibodies. While rodent, particularly murine, neoplastic B cells are preferred, other mammalian species may be employed, such as lagomorpha, bovine, ovine, equine, porcine, avian or the like, which animals and birds can provide lymphocytes, particularly splenocytes, for fusion and will recognize the various human HLA polymorphisms as antigenic. The monoclonal antibodies (MAbs) may be of any of the classes or subclasses, such as IgM, IgD, IgA, IgG1, IgG2, IgG3 or IgG4, or IgE, or the host equivalent thereof.

The monoclonal antibodies which are selected for HLA typing can have a number of properties of interest depending on how they are to be used. Properties of interest include affinity constants $(Ka)$ of at least $10^6$, usually $10^8$, preferably $10^{10}$ $M^{-1}$. The monoclonal antibodies should retain the same specificity over a wide range of dilution (greater than 5-fold, preferably greater than 10-fold) and should not exhibit cross-reactivity as a function of concentration within this range. However, they may have different useful specificities at concentrations outside this range. For some purposes the property of complement fixation is of interest, particularly for use in assays based on complement mediated lysis. The monoclonal antibodies should be stable upon storage for long periods of time.

For the purposes of this application the degree of specificity of the various MAbs (monoclonal antibodies) will be indicated as follows: "Private" intends binding to a unique determinant on a single alloantigen; "oligotypic" intends binding to a determinant which is found on from 2 to 5 different alloantigens; "supertypic" intends binding to a determinant which is found on greater than 5 different alloantigens; and "framework" intends binding to a determinant found on substantially all alloantigens of a particular species. Depending on the purpose for which HLA typing is being performed (e.g., transplant, paternity, etc.) different panels of monoclonals may be employed.

Of particular interest are those oligotypic or supertypic monoclonal antibodies which react with alleles of the same locus. A panel of two to four of these antibodies can completely define this locus. This low stringency definition may well be sufficient for matching donor and recipient in some transplant situations such as platelet transfusion.

Hybridoma cell lines producing monoclonal antibodies having the following binding specificities are of interest: private -A1, 2, 3, 24; Bw6, 8, 13, 44; DR1, 3, 4, 5, 7, w52, w53; DQw1, w3, and w3.1; oligotypic -(A2, w69); (A2, 28), (A23, 24, 25 (32)), -(A25, 32), (B14, 18, 39, 59), (B17, 27), (DR1, 2, 7), (DR5, 8), (DR4, 5, 13), (DR3, 5, 6, 7), (DQw1, DQ blank), and (DQw2, w3, blank); supertypic -(A1, 11, 23, 24, 25, 26, 36, 68.2), (A2, 3, 28, 29, 30, 31, 33, 68, 69), (B7, 27, 42, 54, 55, 56, 57, 58, 63 (14, 46)), and (B8, 18, 39, 41, 45, 61) (specificities in parentheses indicated binding at a different concentration from that at which the other specificities are bound; "blank" indicates an unassigned specificity known to be distinct from DQw1, 2 and 3). These monoclonal antibodies can find use by themselves or in conjuction with antibodies reported by others, e.g., B27, Ellis et , al., Human Immunology (1982) 5 :49.

Specificities which have not been previously reported as being available for an individual monoclonal antibody are: A1, 24, (A1, 11, 23, 24, 25, 26, 36, 68.2), (A2, 3, 28, 29, 30, 31, 33, 68, 69); -(DR5, 8), (DR4, 5, 13), (DR3, 5, 6, 7), (DQw1, DQ blank), and (DQw2, w3, DQ blank).

In accordance with recombinant DNA technology, the messenger RNAs transcribed from the genes encoding the heavy and light chains of the monoclonal antibodies of this invention, can be isolated by differential cDNA hybridization employing cDNA from Balb/c lymphocytes, other than the clones of this invention. Thus, messenger RNA which does not hybridize will be enriched with messenger RNAs encoding the desired immunoglobulin chain. This process can be repeated so as to further enhance the level of the desired messenger RNA. The subtracted mRNA composition can then be reverse-transcribed to provide for a cDNA mixture enriched with desired sequences. The RNA may by hydrolyzed with alkali or an appropriate RNase, and the single strand of DNA made double-stranded with DNA polymerase 1 and random primers, for example, randomly fragmented calf thymus DNA. The resulting double-stranded DNA can then be cloned by insertion into an appropriate vector including virus vectors, such as the lambda vectors, λdv and Charon, or plasmid vectors, such as pBR322 and pACYC184, etc. Known sequences for the constant regions of the light and heavy chain can be used as probes for identifying those clones having the cDNA gene encoding the desired heavy and light chains. By appropriate manipulation, the genes can be excised from the plasmids, manipulated to remove superfluous DNA upstream from the initiation codon, and introduced into an appropriate vector for transformation of a host for expression of the genes. Conveniently, mammalian hosts can be employed which can properly process the chain so as to join the heavy and light chains to produce an intact immunoglobulin and furthermore, secrete that immunoglobulin free of the leader sequence. Alternatively, one can use unicellular microorganisms for producing the chain, where further manipulation may be required to remove the DNA sequence encoding the secretory leader and processing signal, including the membrane integration sequence while providing for an initiation codon at the 5' terminus of the sequence coding for the heavy chain. In this manner the immunoglobulins can be prepared and processed so as to be assembled and glycosylated in cells other than murine cells. If desired, each of the chains can be truncated so as to retain at least the variable region, which regions can then be manipulated to provide for a receptor specific for the HLA polymorphic determinants of interest.

The monoclonal antibodies of this invention find a wide variety of uses, both in vitro and in vivo. In vitro, panels of these antibodies are particularly useful for histocompatibility typing. See, for example, U.S. Patent No. 4,471,056 which is incorporated herein by reference. For such purposes they may be employed in labeled or unlabeled form. When unlabeled they can be used in agglutination or complement-dependent cytotoxicity assays, or in combination with other labeled antibodies specific for immunoglobulin of the particular host species in which the monoclonal antibody was made, or such other immunoassays as involve unlabeled antibodies. Where labeled, a wide variety of labels can be employed, including radionuclides, fluorescers, enzymes, enzyme substrates, enzyme co-factors, enzyme inhibitors, etc. A wide variety of immunoassays are known in the art and are illustrated by the following U.S. patents: 3,817,837; 3,850,752; 3,901,654; 3,935,074; 3,984,533; 3,996,345; 4,034,074; and 4,098,876 (which list is not intended to be exhaustive). The antibodies can be used in a variety of instrumentation, including flow microfluorimeters, where fluorescently conjugated antibodies can be used, where cells can be counted and/or sorted as to their histocompatibility types. Fluorescers of interest include fluorescein, Texas red, rhodamine, umbelliferone, phycobiliproteins, dansyl and the like.

Alternatively, other assays can be used, such as enzyme immunoassays where the subject antibodies are conjugated to an enzyme, or anti-Ig from a species other than the species of the subject antibodies is conjugated to an enzyme, and used in conjunction with the subject antibodies. Instead of anti-Ig a receptor specific for the immune complex may be employed, such as $\underline{S}$. $\underline{aureus}$ protein A. Thus, a human blood sample or a fraction or lysate thereof, is combined with the subject antibodies where the subject antibodies bind to those cells having the HLA alloantigen(s) of interest. The cells can then be separated from the supernatant and incubated with anti-Ig conjugated with an enzyme label, such as horseradish peroxidase (HRP), glucose oxidase (GO), glucose-6-phosphate dehydrogenase (G-6-PDH) or the like. After washing to remove unbound labeled anti-Ig, the appropriate substrates are then added and the presence of the antibody/enzyme conjugates specifically bound to the cells determined. Other conventional techniques can also be used.

An assay of particular interest for HLA typing can be referred to as a Micro-EIA. Microtray wells are coated with peptide, e.g., poly-L-lysine by incubating in a solution of the peptide at a moderately elevated temperature (30-50°C) for about 0.1 to 2hr, the solution decanted and the wells washed. Human leukocytes are introduced into the wells, the trays centrifuged and a dilute buffered protein solution, e.g., 1% BSA or 1.25% powdered milk, added and the trays stored in a cold room, e.g., 4°C, for from 0.25 to 48hr. The plates are then thoroughly washed.

In a mode referred to as indirect, monoclonal antibody to the antigen is added, the mixtures incubated, followed by thorough washing to remove non-specifically bound antibody. Antibody to the monoclonal antibody (anti-IgX, where X is usually M or G), particularly as F(ab')₂, conjugated to an enzyme label is added followed by incubation at ambient temperatures. Usually, 0.2 to 2 hr incubation will suffice. Alternatively, both monoclonal antibody and anti-IgX may be mixed and added together. For the direct assay, the monoclonal antibody is conjugated to the enzyme label, avoiding the addition of the anti-IgX. The labeled antibodies are employed at appropriate concentrations in an appropriately buffered medium with a blocking agent, e.g., 0.1% BSA in PBS.

The antibodies may be grouped into panels depending upon the purpose of histocompatibility typing. Where platelet matching is involved a panel of supertypic and oligotypic antibodies which covers all of the alleles of the A and B gene loci may be sufficient. Thus the antibodies P5.4 and P16.1 and antibodies covering Bw4 (MAb P85.1) and Bw6 (MAb P38.1 and P77.1) could be employed.

In paternity testing, a panel of 25-30 monoclonal antibodies covering the HLA-A and -B alleles would enable one to exclude paternity with a certainty of greater than 90% (see, for example, Family Law Quarterly, $\underline{10}$:(3), 1976, and Jeannet $\underline{et}$ $\underline{al}$., $\underline{Vox}$ $\underline{Sang.}$ (1972) $\underline{23}$:197). For transplantation, panels of antisera would be used to type for both class I (HLA-A,B) and class II (HLA-DR, DQ). For certain disease susceptibility markers, e.g., HLA-associated diseases, such as juvenile onset diabetes, a small panel of antibodies to HLA-DR and DQ could suffice, for example, as few as four. Thus the size of the antibody panel can be tailored to a specific application.

Kits can be supplied for use with the subject antibodies for histocompatibility typing. Thus, the subject monoclonal antibodies can be provided usually as lyophilized compositions by themselves or in conjunction with other antibodies. Incorporated with the antibodies, which can be conjugated to a label, or unconjugated, may be buffers such as TRIS, phosphate, carbonate, borate, etc., stabilizers, biocides, inert proteins, such as bovine serum albumin or the like. Generally these materials are present in less than about 5 weight percent (based on active antibody), and usually present in total amount in at least about 0.001 weight percent - (based on active antibody). Frequently it will be desirable to have present an inert extender or excipient to dilute the active ingredients, where the excipient can be present in from about 1-99 weight percent of the total composition. Where a second antibody is employed which binds to the subject antibodies, this antibody would be conjugated to a label and formulated in an analogous manner with the formulation of the subject antibodies.

The subject antibodies find use in other ways, such as affinity chromatography, purification of the various HLA polymorphic antigens, separation of cells containing one antigen to the exclusion of cells containing other antigens, which separation can involve cytotoxic conditions and the like. Thus, the subject antibodies find a broad spectrum of uses both in vitro and in vivo.

The following examples are offered by way of illustration and not by way of limitation.

EXPERIMENTAL

Materials and Methods

Monoclonal antibody production

Mice of the strains Balb/c, C57B1/6 or $F_1$ progeny thereof are immunized with HLA antigens either as presented on human lymphoblastoid cells or in fractions enriched by immunoprecipitation or HLA antigens obtained from such cells. Mice are immunized by injection subcutaneously or intraperitoneally with or without adjuvant. Mice receive from one to five injections; spleens are removed three days following the last injection.

Splenic B lymphocytes from the immunized mice are fused with NS1-1 or SPO/2 myeloma cells using 40% (w/v) polyethylene glycol (Kodak, MW 1450) and a standard protocol as described (Kohler and Milstein, supra). Following fusion the cell mixture is resuspended in HAT medium (RPMI-1640 medium supplemented with 20% bovine serum, $1 \times 10^{-4}$M hypoxanthine, $4 \times 10^{-7}$M aminopterin and $1.6 \times 10^{-5}$M thymidine) to select for growth of hybrid cells and dispensed into a 96-well microculture tray at a concentration of 1 to $3 \times 10^6$ cells/ml.

Cells producing appropriate antibody are identified either by an assay measuring complement-dependent lysis of human leukocytes ( NAIAD Manual of Tissue Typing Techniques: NIH Lymphocyte Microcytotoxicity Technique USDHEW No. (NIH) 36:41 (1979-1980)) or by an enzyme-linked microimmunoassay measuring binding to human leukocytes (see below). Each hybridoma line is established by three cycles of cloning by limiting dilution using syngeneic thymocytes as feeder cells.

Monoclonal antibody protein is isolated by conventional means from ascites fluid using affinity chromatography on S. aureus protein A sepharose (Pharmacia), ammonium sulphate precipitation or ion exchange chromatography. Ascites fluid is obtained by growth of hybridoma cells in the peritoneal cavity of syngeneic or $F_1$ mice primed one to four weeks earlier with an intraperitoneal injection of pristane. The isotype of the monoclonal antibody is determined by ELISA.

Cell panel

Specificity of the monoclonal antibodies is determined by assaying reactivity to members of a panel of T lymphocytes from 150 individuals and a panel of 160 B lymphoblastoid lines. T lymphocytes are obtained from peripheral blood by density gradient centrifugation on Ficoll-Hypaque, adherence on plastic and passage through nylon wool and B lymphocytes are obtained by eluting adherent cells from the nylon wool (Danilovs et al., 8th International Histocompatibility Workshop Newsletter (1978) 6:3). B lymphoblastoid lines were obtained from peripheral B lymphocytes transformed with Epstein-Barr virus. In addition, each antibody was assayed for specificity and sensitivity on a panel of 250 to 1000 randomly chosen donors.

Micro-enzyme-linked immunosorbent assay (EIA) to detect monoclonal antibody binding to HLA antigens

This assay is used in an indirect mode to identify hybridomas secreting antibody to HLA antigens. This assay may also be used in a direct or an indirect mode with known monoclonal antibodies to determine the HLA type of human cells.

Terasaki microtrays are prepared by addition to each well of 5μl of a 1μg/ml solution of poly-L-lysine in phosphate buffered saline (PBS). Alternatively, antibodies to human leukocytes (5μl of 0.1μg per μl) may be added to each well and the plates incubated at 40°C for 18-24 hours.

The plates are incubated at 37°C for 1hr and washed with PBS by immersion and decanting. Human leukocytes are dispensed into each well, 1μl of a suspension of 1 to $5 \times 10^6$ cells per ml of RPMI-1640 medium without serum. The plates are centrifuged at 90g for 3min. A solution of 1% bovine serum albumin (BSA) in PBS with 0.2% azide is added to the plates which are stored at 4°C for 1 to 48 hours or at ambient temperature for 15 minutes. Before adding antibody, the plates are washed with PBS and 1.25% powdered milk in PBS.

In the indirect assay, monoclonal antibody is added, 1μl per well. After 1 hour at room temperature, the plates are washed five times and a solution of the $F(ab')_2$ fragment of anti-immunoglobulin coupled with horseradish peroxidase (HRP) is added, 5μl per well. Alternatively, both monoclonal antibody and anti-immunoglobulin may be mixed and added together. The plates are then incubated at room temperature for 30 to 60min. In the direct assay, HRP is coupled to the monoclonal antibody, the second step is thus unnecessary. Antibodies coupled to HRP are diluted in a solution of 0.1% BSA in PBS without azide.

After treatment with antibody, the trays are washed five times. The presence of HRP-antibody complexes in the wells is visualized by the addition of a solution of substrate, hydrogen peroxide, and chromagen, OPD (Organon Diagnostics, West Orange, NJ) or ABTS (Boehringer-Mannheim Biochemicals, Indianapolis, IN) in 0.1M sodium citrate/0.2M sodium phosphate. Color change in wells after 30 to 60min incubation at room temperature indicates binding of monoclonal antibody to leukocytes in those wells.

Analysis of the specificity of monoclonal antibodies by immunoprecipitation of labeled HLA antigens

Membrane proteins of lymphoblastoid cells are labeled with $^{125}I$ using lactoperoxidase (Vitetta et al., J. Exp. Med. (1971) 134:242). Cells are disrupted in buffer containing 0.5% Nonidet P-40 and cleared by centrifugation both before and after addition of control antibody and solid phase immunoadsorbent. Monoclonal antibodies are added to aliquots of the lysate and complexes of antibody and labeled antigen precipitated using S. aureus protein A or rabbit antibody to mouse immunoglobulin coupled to a solid phase. After extensive washing, the labeled antigens are released by addition of sample electrophoresis buffer either as required for the Laemmli method of electrophoresis in polyacrylamide gels or as specified for the non-equilibrium two-dimensional gel electrophoresis method of O'Farrell. Electrophoresis is conducted as required for each method. Labeled proteins in the dried gels are identified by radioautography.

Analysis of the specificity of monoclonal antibodies labeled with fluorescein isothiocyanate (FITC) using a fluorescence-activated cell sorter (FACS)

Monoclonal antibody isolated from ascites was conjugated to FITC according to the method of Goding (Goding, J. Immunol. Methods (1976) 13:215). Cells to be analyzed were mixed with saturating amounts of FITC-conjugated antibody and incubated for 30min at 4°C. Treated cells were washed and the amount of antibody bound assessed by comparing the fluorescence intensity - (mean modal) of cells incubated with test and control antibodies on a FACS IV (Becton-Dickinson) fitted with a log amplifier.

Results

A small proportion of the total number of fusions provided murine monoclonal antibodies which are specific for HLA epitopes which are present on one or a limited number of class I or class II alleles. A list of clones producing antibody specific for HLA allodeterminants is present in Tables 1 - (class I) and 2 (class II).

TABLE 1:  MONOCLONAL ANTIBODIES FOR A CLASS I PANEL

| Antibody | Isotype | Specificity | ATCC Accession No. |
|---|---|---|---|
| GSP5.1 | IgM | A2,w69 | HB 8820 |
| GSP5.3 | IgG$_{2a}$ | B7,27,42,54,55,56,57,58,63(14,46) | HB 8818 |
| GSP5.4 | IgG$_{2a}$ | A2,3,28,29,30,31,33,68,69 | HB 8819 |
| GSP7.1 | IgG$_{2a}$ | B7,27,42,54,55,56,57,58,63(14,46) | HB 8817 |
| GSP8.1 | IgG$_{2b}$ | B8 | HB 8671 |
| GSP10.1 | IgG$_{2b}$ | All Class I | - |
| GSP16.1 | IgM | A1,11,23,24,25,26,36,68.2 | HB 8816 |
| GSP17.2 | IgM | B8,18,39,41,45,61 | HB 8815 |
| GSP20.1 | IgG$_{2b}$ | A2,w69 | HB 8813 |
| GSP35.1 | IgM | A2,28 | HB 8814 |
| GSP38.1 | IgG$_{2b}$ | Bw6 | HB 8838 |
| GSP49.2 | IgM | B13 | HB 8812 |
| GSP55.1 | IgG$_{2a}$ | A25,32 | HB 8809 |
| GSP55.2 | IgG$_{2b}$ | A3 | HB 8811 |
| GSP56.1 | IgM | A32,B27 | HB 8810 |
| GSP74.1 | IgM | A29 | HB 8829 |
| GSP77.1 | IgM | Bw6 | HB 8827 |
| GSP81.2 | IgG$_{2a}$ | A3 | HB 8828 |
| GSP84.1 | IgM | A25,32 | HB 8826 |
| GSP84.2 | IgG$_{2a}$ | B14,18,39,59 | HB 8824 |
| GSP85.1 | IgM | A23,24,25,32,B27,38,49,52,53,57,59,63 | HB 8855 |
| GS112.1 | IgM | A23,24,25(32) | HB 8841 |
| GS114.1 | IgM | A24 | HB 8844 |
| GS129.5 | IgM | B17,27 | HB 8825 |
| GS142.1 | IgG$_{2a}$ | A1 | HB 8843 |
| GS145.2 | IgG$_1$ | B27 | HB 8856 |
| GS157.2 | IgG$_{2b}$ | A2 | - |
| GS159.3 | IgG$_1$ | B44 | - |

TABLE 2: MONOCLONAL ANTIBODIES FOR A CLASS II PANEL

| Antibody | Isotype | Specificity | ATCC Accession No. |
|---|---|---|---|
| GSP 4.1 | $IgG_{2a}$ | All DR | μβ 8821 |
| GSP 14.1 | $IgG_{2b}$ | DR other than DR7 | μβ 8842 |
| GSP 17.1 | IgM | DQw2, w3, blank | μβ 8853 |
| GSP 65.1 | IgM | DR7 | μβ 8839 |
| GSP 88.2 | IgM | DR5 | μβ 8840 |
| GSP 91.1 | IgM | DR5, 8 | a |
| GSP 97.1 | $IgG_{2b}$ | DRw53 | a |
| GS 100.1 | IgM | DQw3 | μβ 8822 |
| GS 103.1 | IgM | DR 4, 5, 13 | a |
| GS 105.2 | IgM | DR 3, 5, 6, 7 | a |
| GS 109.1 | IgM | DQw3 | a |
| GS 142.1 | $IgG_{2a}$ | DRw52 | μβ 8845 |
| GS 143.1 | IgA | DR3 | μβ 8854 |
| GS 147.1 | IgG | DR 1, 2, 7 | a |
| GS 148.1 | IgM | DQw1 | a |
| GS 158.1 | $IgG_{2b}$ | DQw2, w3, blank | a |
| GS 159.2 | $IgG_1$ | DQw3.1 | ATCC pending μβ9113 |
| GS 175.1 | $IgG_{2b}$ | DQw1 | a |
| GS 179.1 | $IgG_1$ | DR4 (not Dw13), 7 | a |
| GS 192.1 | ND | DR4 (Dw13 only) | a |
| GS 200.1 | ND | DQw3.2 | ATCC pending μβ9111 |
| GS 210.1 | ND | DR1 | ATCC pending μβ9112 |
| GS 4AA7 | $IgG_1$ | DQw1, blank | μβ 8823 |

a - Deposited at HLA Laboratory, Puget Sound Blood Center, Seattle, Washington.
ND - not determined

Examples of how a number of these antibodies can be combined in panels to determine the HLA type of T or B lymphocytes are presented in the following Tables 3, 4 and 5.

TABLE 3:    TYPING FOR HLA-A LOCUS SPECIFICITIES USING A PANEL
OF MONOCLONAL ANTIBODIES

T lymphocytes       Monoclonal Antibodies

| Donor | HLA-A | 5.4 | 16.1 | 20.1 | 55.2 | 74.1 | 114.1 | 142.1 | 112.1 | 55.1 | 56.1 |
|-------|-------|-----|------|------|------|------|-------|-------|-------|------|------|
| 049 | 3,26 | + | + | − | + | − | − | − | − | − | − |
| 051 | 3,25 | + | + | − | + | − | − | − | + | + | − |
| 074 | 3,29 | + | − | − | + | + | − | − | − | − | − |
| 070 | 2,24 | + | + | + | − | − | + | − | + | − | − |
| 079 | 2,32 | + | − | + | − | − | − | − | − | + | + |
| 052 | 25,29 | + | + | − | − | + | − | − | + | + | − |
| 058 | 1,32 | − | + | − | − | − | − | − | − | + | + |

TABLE 4:    TYPING B LYMPHOCYTE CELL LINES FOR HLA CLASS II
SPECIFICITIES USING A PANEL OF MONOCLONAL ANTIBODIES

B lymphocyte lines       Monoclonal Antibodies

| Name | HLA-DR | HLA-DQ | 4.1 | 14.1 | 65.1 | 88.2 | 143.1 | 4AA7 | 17.1 | 100.1 |
|------|--------|--------|-----|------|------|------|-------|------|------|-------|
| MAT | 3 | 2 | + | + | − | − | + | − | + | − |
| MYR | 3 | 1 | + | + | − | − | + | + | − | − |
| SWEI | 5 | 3 | + | + | − | + | − | − | + | + |
| SOM | 5 | 1 | + | + | − | + | − | + | − | − |
| JB | 7 | 2 | + | − | + | − | − | − | + | − |
| JK | 7 | 3 | + | − | + | − | − | − | + | + |

TYPING FOR HLA CLASS II SPECIFICITIES USING A PANEL
OF MONOCLONAL ANTIBODIES ON B LYMPHOCYTES

MONOCLONAL ANTIBODIES

B LYMPHOCYTES

| Donor | HLA-DR | HLA-DQ | 65.1 | 88.2 | 143.1 | 210.1 | 179.1 | 142.1 | 97.1 | 175.1 | 158.1 | 100.1 |
|---|---|---|---|---|---|---|---|---|---|---|---|---|
| 099 | 3,7,52,53 | 2 | + | - | + | - | + | + | + | - | + | - |
| 011 | 1,5,52 | 1,3 | - | + | - | + | - | + | - | + | + | + |
| 100 | 1,4,53 | 1,3 | - | - | - | + | + | - | + | + | + | + |
| 079 | 5,7,52,53 | 3 | + | + | - | - | + | + | + | - | + | + |
| 017 | 1,5,52 | 1,3 | - | + | - | + | - | + | - | - | + | + |

0 204 522

Methods used in typing for HLA specificities

There are three general kinds of assays used to type human lymphocytes for HLA antigen expression. First, and the one most used at the present time, is complement-dependent lysis. Second are assays measuring binding of antibody to lymphocytes by enzyme-linked immunoassay - (ELISA). Elements of the first method have been combined with standard format ELISA assays to develop a micro ELISA assay suitable for HLA typing. The third uses a fluorescing compound to measure binding of antibody to lymphocytes. Fluorescein isothiocyanate and a fluorescence-activated cell sorter was used to analyze the specificity of the subject antibodies.

All antibodies listed in Table 1 have been tested and give the same reactions in each of these three assays. An example of typing human T lymphocytes with a panel of these antibodies is given in Table 3. The antibodies in Table 2 also are effective in the three assay systems except for antibodies 4AA7, 143.1 and 142.1; these antibodies do not fix complement and are suitable for assays 2 and 3 only. An example of typing B lymphocytes for class II HLA antigens using several monoclonal antibodies is given in Table 4.

The panels of Tables 3 and 4 consist of a series of HLA monoclonal antibodies which are chosen to define a series of overlapping but distinct HLA antigens. The matrix of positive and negative results with the panel of antibodies permits an assignment of an HLA type in the tested individual.

A panel of monoclonal antibodies has several advantages over previously available conventional alloantisera panels: (1) these antibodies are available in infinite supply, thus, once their specificity is defined no additional characterization is required. By contrast, tissue typing is currently carried out using sera obtained from multiparous women or multiply-transfused patients. Only limited amounts of such reagents are available from any one donor. Accordingly, it is necessary to continually replace these antisera with new reagents which must be standardized and checked against the previous ones. (2) Because of the heterogeneous nature of antibodies obtained in this fashion, cross-reactivity and contamination with multiple antibodies of varying specificity are major problems. With a monoclonal panel, individual cloned antibodies can be used at high concentration without concern for contaminating specificities. (3) Alloantisera are usually very low titered thus requiring a higher concentration of serum in the reaction mixture. This can contribute to non-specific binding of protein to cell surfaces and result in false negative results in the complement-dependent assay. Furthermore, these sera are less useful in binding assays due to the potential of nonspecific binding of protein to cell surface. Monoclonal antibodies in contrast can be purified and concentrated to produce titers in the thousands. (4) Because of the exquisite specificity of these antibodies they have wider application in a variety of assay formats. The use of the monoclonal antibody panel makes possible the use of a variety of assay methodologies which are not feasible with the conventional antibody panels. These include, but are not limited to, automated fluorescence methods, ELISA-based methods, and flow cytometry.

Although the foregoing invention has been described in some detail by way of illustration and example for purposes of clarity of understanding, it will be obvious that certain changes and modifications may be practiced.

## Claims

1. A monoclonal antibody composition having the same antibody specificity as one of the monoclonal antibodies designated as 5.4, 16.1, 17.1, 114.1, 142.1, 145.2, 159.2, 200.1, 210.1 and 4AA7.

2. A histocompatibility typing panel comprising at least two monoclonal antibodies which distinguish between different alloantigens and having at least one monoclonal antibody according to Claim 1.

3. A panel according to Claim 2, wherein said monoclonal antibody is murine.

4. A monoclonal antibody according to Claim 1, wherein said monoclonal antibody is murine.

5. A monoclonal antibody according to Claim 1, wherein said antibodies are of the class IgM or IgG.

6. A monoclonal antibody according to Claim 1, conjugated to a label capable of providing a detectable signal.

7. A monoclonal antibody according to Claim 6, wherein said label is a fluorescer or an enzyme.

8. A monoclonal antibody consisting of a monoclonal antibody listed in Table 1 or Table 2.

9. A histocompatibility typing panel comprising monoclonal antibodies which distinguish between different alloantigens and having at least two monoclonal antibodies according to Claim 8.

10. A monoclonal antibody according to Claim 8, conjugated to a label capable of providing a detectable signal.

11. A monoclonal antibody according to Claim 10, wherein said label is a fluorescer or enzyme.

12. A method for HLA typing which comprises combining human cells with a monoclonal antibody according to any of Claims 8 or 10 and detecting complex formation.

13. A method according to Claim 12, including the steps of:

(1) introducing a human leukocyte suspension into wells coated to enhance binding;

(2) centrifuging said cell containing wells;

(3) removing supernatant and washing said cell containing wells;

(4) adding monoclonal antibody specific for an alloantigen into at least one well, wherein different monoclonal antibodies are put into different wells; and

(5) washing said wells to remove non-specifically bound monoclonal antibody, wherein said complex formation is detected by employing monoclonal antibody conjugated with an enzyme or by adding a receptor conjugated to an enzyme which binds to said complex, and detecting the presence of specifically bound enzyme.

14. A method according to Claim 12, wherein said monoclonal antibody is of a subclass which permits complement mediated lysis and complex formation is detected by complement mediated lysis.

15. A kit for use in the method according to Claim 12, comprising at least one monoclonal antibody present in Tables 1 or 2 conjugated to a label providing for a detectable signal or said kit including an antibody to said monoclonal antibody composition conjugated to a label providing for a detectable signal.

16. A murine gene encoding for at least one chain of monoclonal antibody of a composition according to Claim 1 in a host capable of in vitro culture.